# EUROPÄISCHE PATENTSCHRIFT

(11) **EP 1 871 707 B1**
(45) Veröffentlichungstag und Bekanntmachung des Hinweises auf die Patenterteilung: **15.10.2008**
(21) Anmeldenummer: 06725089.4
(22) Anmeldetag: 16.03.2006
(51) Int. Cl.: C01B 15/029, C07D 301/12

(54) **VERFAHREN ZUR HERSTELLUNG VON WASSERSTOFFPEROXID**
METHOD FOR PRODUCTION OF HYDROGEN PEROXIDE
PROCEDE DE FABRICATION DE PEROXYDE D'HYDROGENE

(30) Priorität: 13.04.2005 DE 102005016877; 12.04.2005 US 670229 P
(43) Veröffentlichungstag der Anmeldung: 02.01.2008
(73) Patentinhaber: Evonik Degussa GmbH, 45128 Essen (DE)
(72) Erfinder: JAHN, Robert, 60322 Frankfurt (DE); HAAS, Thomas, 48161 Münster (DE)
(86) Internationale Anmeldenummer: PCT/EP2006/060775
(87) Internationale Veröffentlichungsnummer: WO 2006/108748

(56) Entgegenhaltungen:
- EP-A- 0 504 741
- EP-A- 0 987 316
- EP-A- 1 344 747
- US-A- 6 143 688
- US-A1- 2003 083 510

## Beschreibung

Die Erfindung richtet sich auf ein Verfahren zur Herstellung von Wasserstoffperoxid durch Reaktion von Wasserstoff und Sauerstoff in Gegenwart eines Edelmetallkatalysators in einem flüssigen Reaktionsmedium, wobei die Reaktion in Gegenwart eines Schwefelsäurealkylesters durchgeführt wird, sowie auf nach diesem Verfahren erhältliche Lösungen von Wasserstoffperoxid in Methanol und deren Verwendung zur Epoxidierung von Olefinen.

Es ist bekannt, Wasserstoffperoxid durch Direktsynthese aus Wasserstoff und Sauerstoff enthaltenden Gasgemischen herzustellen, indem das Gasgemisch in Gegenwart eines flüssigen wässrigen, wässrig-organischen oder organischen Reaktionsmediums an einem Edelmetallkatalysator umgesetzt wird. Die nach dem Direktsyntheseverfahren erhältlichen Wasserstoffperoxidlösungen sind von Interesse als Oxidationsmittel für die katalytische Oxidation von organischen Verbindungen.

Problematisch ist bei der Direktsynthese von Wasserstoffperoxid die unerwünschte katalytische Aktivität der verwendeten Edelmetallkatalysatoren in der katalytischen Zersetzung von Wasserstoffperoxid zu Wasser und Sauerstoff. Diese unerwünschte Aktivität des Katalysators lässt sich hemmen, indem dem flüssigen Reaktionsmedium eine starke Säure und ein Halogenid in einer ausreichenden Konzentration zugesetzt werden. Durch den für die Erzielung einer hohen Wasserstoffperoxid-Selektivität erforderlichen Zusatz an Säure und Halogenid wird das flüssige Reaktionsmedium allerdings stark korrosiv gegenüber metallischen Werkstoffen, insbesondere gegenüber Edelstahl. Außerdem führt insbesondere der Gehalt an Säure bei der Verwendung der Wasserstoffperoxidlösung als Oxidationsmittel für die katalytische Oxidation von organischen Verbindungen zu unerwünschten Nebenreaktionen und Folgereaktionen.

Ein Ansatz zur Lösung des Problems ist die Verwendung von stark sauren Katalysatorträgern, wie sauren und supersauren Metalloxiden, bekannt aus EP 504 741, Aktivkohlen mit Sulfonsäuregruppen, bekannt aus EP 978 316, oder Ionenaustauscherharzen mit sauren Gruppen, bekannt aus EP 1 344 747. Edelmetallkatalysatoren auf stark sauren Katalysatorträgern haben jedoch den praktischen Nachteil, dass der Katalysator bei der Herstellung von Wasserstoffperoxid rasch einen großen Teil der katalytischen Aktivität verliert.

Deshalb besteht weiterhin ein Bedarf an verbesserten Verfahren zur Direktsynthese von Wasserstoffperoxid, die eine hohe Selektivität der Wasserstoffperoxidbildung bei anhaltend hoher Aktivität des Katalysators auch ohne Zusatz von Säure oder bei nur geringen Säurekonzentrationen gewährleisten.

Es wurde nun überraschend gefunden, dass bei der Direktsynthese von Wasserstoffperoxid an einem Edelmetallkatalysator in Gegenwart eines Schwefelsäurealkylesters eine höhere Aktivität und Selektivität des Katalysators für die Wasserstoffperoxidbildung erzielt wird, ohne dass der Katalysator dabei an Aktivität verliert. In Gegenwart eines Schwefelsäurealkylesters kann auch mit verringerter Säuremenge oder ohne Säurezusatz eine hohe Selektivität der Wasserstoffperoxidbildung erzielt werden.

Gegenstand der Erfindung ist deshalb ein Verfahren zur Herstellung von Wasserstoffperoxid durch Reaktion von Wasserstoff und Sauerstoff in Gegenwart eines Edelmetallkatalysators in einem flüssigen Reaktionsmedium, dadurch gekennzeichnet, dass die Reaktion in Gegenwart eines Schwefelsäurealkylesters durchgeführt wird.

Gegenstand der Erfindung ist außerdem eine nach diesem Verfahren erhältliche Wasserstoffperoxidlösung, enthaltend von 2 bis 15 Gew.-% Wasserstoffperoxid,
von 0,5 bis 20 Gew.-% Wasser,
von 60 bis 95 Gew.-% Methanol,
von 10⁻⁶ bis 10⁻² mol/l Bromid und
von 10⁻⁶ bis 0,1 mol/l Dimethylsulfat und/oder Monomethylsulfat

Gegenstand der Erfindung ist weiterhin ein Verfahren zur Herstellung von Epoxiden durch Umsetzung eines Olefins mit Wasserstoffperoxid in Gegenwart eines titanhaltigen Zeoliths als Katalysator unter Einsatz der erfindungsgemäßen Wasserstoffperoxidlösung.

Bei dem erfindungsgemäßen Verfahren zur Herstellung von Wasserstoffperoxid werden Wasserstoff und Sauerstoff in Gegenwart eines Edelmetallkatalysators in einem flüssigen Reaktionsmedium umgesetzt, das einen Schwefelsäurealkylester enthält. Schwefelsäurealkylester im Sinne der Erfindung sind die Produkte der Veresterung von Schwefelsäure mit einem aliphatischen Alkohol. Geeignet sind dabei sowohl Schwefelsäuredialkylester als auch Schwefelsäuremonoalkylester, wobei Schwefelsäuremonoalkylester auch in Form der Alkalimetall- oder Ammoniumsalze eingesetzt werden können. Vorzugsweise werden Schwefelsäurealkylester eingesetzt, deren Alkylrest aus der Gruppe Methyl, Ethyl, n-Propyl und n-Butyl ausgewählt ist. Besonders bevorzugt sind Schwefelsäurealkylester mit Methyl als Alkylrest. Für das erfindungsgemäße Verfahren eignen sich insbesondere Dimethylsulfat, Diethylsulfat, Schwefelsäuremonomethylester, Ammoniummethylsulfat, Natriummethylsulfat und Kaliummethylsulfat.

Die Menge an Schwefelsäurealkylester im flüssigen Reaktionsmedium wird entsprechend den anderen Komponenten des flüssigen Reaktionsmediums so gewählt, dass eine ausreichende Aktivität und Selektivität des Katalysators erzielt wird und liegt vorzugsweise im Bereich von 10⁻⁶ mol/l bis 0,1 mol/l und besonders bevorzugt im Bereich von 0,001 mol/l bis 0,1 mol/l. Liegt die Konzentration an Schwefelsäurealkylester unterhalb des bevorzugten Bereichs, dann wird in der Regel zum Erreichen einer ausreichenden Selektivität der Zusatz einer starken Säure erforderlich, während in Gegenwart eines Schwefelsäurealkylester im bevorzugten Konzentrationsbereich auch ohne Zusatz einer starken Säure gearbeitet werden kann.

Das flüssige Reaktionsmedium kann ein wässriges, wässrigorganisches oder organisches Reaktionsmedium sein. Bei Verwendung eines wässrig-organischen oder organischen Reaktionsmediums wird als organische Komponente vorzugsweise ein alkoholisches Lösungsmittel eingesetzt, wobei primäre Alkohole besonders bevorzugt sind. Vorzugsweise wird ein Lösungsmittel aus der Reihe Methanol, Ethanol, n-Propanol und n-Butanol und besonders bevorzugt Methanol eingesetzt. Im Hinblick auf eine Verwendung der Wasserstoffperoxidlösung als Oxidationsmittel zur Oxidation organischer Verbindungen ist es zweckmäßig, wenn der Wassergehalt im Reaktionsmedium nach Möglichkeit niedrig gehalten wird. Zweckmäßigerweise wird der Wassergehalt im Reaktionsmedium auf maximal 20 Gew.-%, vorzugsweise 10 Gew.-% begrenzt. Besonders bevorzugt wird als Lösungsmittel Methanol mit einem Wassergehalt von 2 bis 10 Gew.-% verwendet.

Das zur Direktsynthese verwendete flüssige Reaktionsmedium enthält vorzugsweise noch ein Halogenid gelöst in einer Menge, die geeignet ist, um die Zersetzung von Wasserstoffperoxid am Edelmetallkatalysator während der Direktsynthese zu hemmen. Als Halogenide werden Bromid und/oder Iodid und bevorzugt Bromid verwendet. Das flüssige Reaktionsmedium enthält das Halogenid vorzugsweise in einer Konzentration im Bereich von 10⁻⁶ bis 10⁻² mol/l, besonders bevorzugt im Bereich von 10⁻⁵ bis 10⁻³ mol/l und insbesondere im Bereich von 10⁻⁵ bis 5.10⁻⁴ mol/1. Liegt die Halogenidkonzentration oberhalb des bevorzugten Bereichs, dann wird die Stabilität der hergestellten Wasserstoffperoxidlösung beeinträchtigt, liegt sie unterhalb des bevorzugten Bereichs, dann wird in der Regel nicht mehr eine ausreichende Wasserstoffperoxid-Selektivität erreicht. Niedrigere Halogenidkonzentrationen werden im Hinblick auf die Weiterverwendung der gebildeten Wasserstoffperoxidlösung bevorzugt. Das Halogenid kann dem Reaktionsmedium in Form eines Alkalimetall- oder Erdalkalimetallsalzes zugesetzt werden, bevorzugt als NaBr oder NaI. Ebenso kann das Halogenid auch in Form der Halogenwasserstoffsäure, zum Beispiel als HBr oder HI zugesetzt werden.

Das zur Direktsynthese verwendete flüssige Reaktionsmedium kann gegebenenfalls zusätzlich noch eine starke Säure enthalten. Starke Säuren im Sinn der Erfindung sind dabei alle Säuren, die einen pKₐ-Wert von weniger als 3 und vorzugsweise einen pKₐ-Wert von weniger als 2 aufweisen. Geeignet sind insbesondere Mineralsäuren, wie Schwefelsäure, Phosphorsäure und Salpetersäure. Ebenso einsetzbar sind auch im Medium lösliche Sulfonsäuren und Phosphonsäuren. Die Säurekonzentration im organischen oder organisch-wässrigen flüssigen Medium liegt vorzugsweise im Bereich von 0,0001 bis 0,5 mol/l und besonders bevorzugt im Bereich von 0,001 bis 0,1 mol/l. Liegt die Säurekonzentration oberhalb des bevorzugten Bereichs, dann wird die Flüssigphase unerwünscht korrosiv, liegt sie unterhalb des bevorzugten Bereichs, dann kann es bei niedrigen Konzentrationen an Schwefelsäurealkylester zu einer Abnahme der Wasserstoffperoxid-Selektivität kommen. Niedrigere Säurekonzentrationen werden im Hinblick auf die Weiterverwendung der gebildeten Wasserstoffperoxidlösung bevorzugt.

Die Reaktion wird vorzugsweise mit einem Wasserstoff und Sauerstoff enthaltenden Gasgemisch durchgeführt, dessen Zusammensetzung so gewählt ist, dass das Gasgemisch nicht explosionsfähig ist. Bevorzugt wird ein Gasgemisch verwendet, das auch unter Berücksichtigung des sich einstellenden Lösungsmittelpartialdrucks zuverlässig außerhalb der Explosionsgrenze liegt. Zweckmäßiger Weise enthält das Gasgemisch neben Wasserstoff und Sauerstoff auch ein oder mehrere Inertgase, vorzugsweise Stickstoff. Der Wasserstoffgehalt im Gasgemisch, wird auf maximal 6 Volumen-% begrenzt, vorzugsweise maximal 5 Volumen-%. Insbesondere liegt der Wasserstoffgehalt im Bereich von 3 bis 5 Volumen-%. Der Sauerstoffgehalt im Gasgemisch kann stöchiometrisch oder überstöchiometrisch sein und liegt vorzugsweise im Bereich von 10 bis 50 Volumen-%, insbesondere 15 bis 45 Volumen-%. Wasserstoff und Sauerstoff werden vorzugsweise getrennt dem Reaktor zugeführt. Sauerstoff kann dabei sowohl in reiner Form, als auch in Form von Luft oder von mit Sauerstoff angereicherter Luft zugeführt werden. Bei einer kontinuierlichen Reaktionsführung kann das am Ausgang des Reaktors erhaltene Restgas ganz oder teilweise in den Reaktor zurückgeführt werden, um den Aufwand für die Rückgewinnung von nicht umgesetztem Wasserstoff zu verringern.

Für das erfindungsgemäße Verfahren können alle für die Direktsynthese von Wasserstoffperoxid aus dem Stand der Technik bekannten, ein oder mehrere Edelmetalle enthaltenden Katalysatoren verwendet werden. Besonders geeignete Katalysatoren sind aus EP-A 1 038 833, Seite 3, Zeile 10 bis Seite 4, Zeile 14, aus US 6,168,775, Spalte 5, Zeile 65 bis Spalte 6, Zeile 64 und aus WO 2005/009611, Seite 34, Zeile 19 bis Seite 42, Zeile 20 bekannt.

Die katalytisch wirksame Komponente des Katalysators enthält ein oder mehrere Edelmetalle in reiner Form oder in Form von Legierungen. Bevorzugte Edelmetalle sind die Platinmetalle, insbesondere Palladium, sowie Gold. Zusätzlich können Elemente aus der Reihe Rh, Ru, Ir, Cu und Ag anwesend sein. Besonders bevorzugte Katalysatoren enthalten als katalytisch wirksame Metalle mindestens 80 Gew.-% Palladium und 0 bis 20 Gew.-% Platin, sowie 0 bis 20 Gew.-% Gold und/oder 0 bis 5 Gew.-% Silber in legierter oder unlegierter Form.

Die Katalysatoren können sowohl trägerfrei, als auch trägergebunden vorliegen, wobei trägergebundene Katalysatoren bevorzugt sind. Das oder die katalytisch wirksamen Edelmetalle können sich auf der Oberfläche eines Trägermaterials befinden und/oder als Partikel in gleichmäßiger Verteilung innerhalb einer Schüttung eines inerten Trägermaterials angeordnet sein.

Bei den Trägermaterialien handelt es sich um partikelförmige Materialien, wie Pulver, Extrudate, Granulate, oder andere aus einem pulverförmigen Material gebildete Formkörper. Vorzugsweise werden oxidische oder silikatische Trägermaterialen verwendet, insbesondere Aluminiumoxid, Kieselsäure, Titandioxid, Zirkoniumdioxid und Zeolithe. Alternativ können auch Träger auf Kohlenstoffbasis, wie zum Beispiel Aktivkohleträger verwendet werden.

Es ist möglich, die in feinstverteilter Form vorliegende katalytische wirksame Komponente mit einem pulverförmigen Trägermaterial zu vermischen, das Gemisch zu plastifizieren, zu verformen und die Formlinge durch eine Kalzination zu verfestigen. Gemäß einer Alternative ist es auch möglich, einen bereits vorgefertigten geformten Träger mit einer die feinstverteilte katalytisch wirksame Komponente enthaltenden Suspension zu imprägnieren, wobei ein sogenannter Schalenkatalysator erhalten wird. Bei der Applizierung des katalytisch wirksamen Materials auf bzw. in dem Trägermaterial können auch bekannte Bindemittel, wie Wasserglas, Calciumoxalat, Borsäure und andere glasbildende Zusammensetzungen, zugegen sein. Üblicherweise schließt sich an das Aufbringen des katalytisch wirksamen Materials auf ein Trägermaterial ein Kalzinierschritt bei 300 bis 600°C an. Schließlich lassen sich die katalytisch wirksamen Trägerkatalysatoren auch durch Imprägnierung des Trägers mit einer Lösung, welche eine Verbindung der katalytisch wirksamen Metalle enthält, und sich anschließende Hydrier-, Kalzinier- und Waschschritte gewinnen.

Das erfindungsgemäße Verfahren kann sowohl kontinuierlich als auch diskontinuierlich durchgeführt werden, wobei eine kontinuierliche Reaktionsführung bevorzugt wird. Der Edelmetallkatalysator kann dabei in beliebiger Form, insbesondere in Form einer Suspension oder in Form eines Festbetts eingesetzt werden.

Vorzugsweise wird der Edelmetallkatalysator in Form eines Festbetts eingesetzt. Die Größe der Partikel im Festbett kann in weiten Bereichen, insbesondere im Bereich von 0,1 bis 10 mm liegen. Bei Verwendung von Mischungen aus katalytisch aktiven und inaktiven Partikeln können auch katalytisch aktive Partikel mit einer Größe im Bereich von 0,02 bis 0,1 mm verwendet werden. Eine geringe Partikelgröße führt zu einem höheren Druckabfall, bei zu großer Partikelgröße nimmt die katalytisch wirksame Oberfläche ab. Partikelgrößen im Bereich von 0,1 bis 5 mm, insbesondere 0,1 bis 2 mm und besonders bevorzugt 0,1 bis 0,5 mm führen zu hohen Produktivitäten.

In einer bevorzugten Ausführungsform des erfindungsgemäßen Verfahrens wird ein Reaktor verwendet, der ein oder mehrere Festbetten mit dem Edelmetallkatalysator enthält. Der Reaktor wird mit dem flüssigen Reaktionsmedium geflutet und das Gasgemisch wird in dem im Reaktor enthaltenen flüssigen Reaktionsmedium in Form von Gasblasen verteilt. Der Reaktor wird dabei so konstruiert und betrieben, dass sich in dem Reaktor keine Gaspolster ausbilden, in denen das Gasgemisch dauerhaft in Kontakt mit der Reaktorwand oder mit Reaktoreinbauten aus Metall steht. Ein Beispiel für eine solche erfindungsgemäße Ausführungsform ist die Durchführung der Reaktion in einer Blasensäule, wobei das flüssige Reaktionsmedium und das Gasgemisch im unteren Teil der Blasensäule zugeführt werden und die hergestellte Wasserstoffperoxidlösung zusammen mit nicht umgesetztem Gas am höchsten Punkt des Blasensäulenreaktors entnommen wird. Der Blasensäulenreaktor enthält dabei ein Festbett aus trägergebunden Katalysatorpartikeln oder einem Gemisch aus katalysatorhaltigen und katalysatorfreien Partikeln, wobei die Einbauten, die das Katalysatorfestbett im Reaktor halten, so ausgebildet sind, dass sich an keiner Stelle ein Gaspolster ausbildet, das dauerhaft in Kontakt mit der Reaktorwand oder mit Einbauten aus Metall steht. Dauerhaft in Kontakt stehen bedeutet dabei, dass die Oberfläche über einen längeren Zeitraum nicht mit dem flüssigen Reaktionsmedium benetzt ist, sondern in unmittelbaren Kontakt mit der Gasphase steht. Vorzugsweise bleibt die Oberfläche an keiner Stelle des Reaktors länger als 30 Minuten vom flüssigen Medium unbenetzt, insbesondere nicht länger als 30 Sekunden. In der am meisten bevorzugten Ausführungsform wird das flüssige Reaktionsmedium so durch den Reaktor geführt, dass die Oberfläche des Reaktors an jeder Stelle ständig von dem flüssigen Rektionsmedium benetzt wird.

Bei Verwendung eines Festbettkatalysators wird das flüssige Reaktionsmedium dem Reaktor vorzugsweise mit einer Rate zugeführt, die im Reaktor zu einer Querschnittsbelastung des Katalysators durch die Flüssigphase im Bereich von 0,3 bis 200 m/h bezogen auf den Leerquerschnitt des Reaktors führt. Vorzugsweise liegt die Querschnittsbelastung im Bereich von 0,3 bis 20 m/h und besonders bevorzugt im Bereich von 1 bis 10 m/h. Im bevorzugten Bereich der Querschnittsbelastung können Wasserstoffperoxidlösungen mit einem Wasserstoffperoxidgehalt von 4 bis 12 Gew.-% hergestellt werden, wobei gleichzeitig eine hohe Wasserstoffperoxid-Selektivität, eine hohe Raum-ZeitAusbeute und eine hohe Katalysatorstandzeit erzielt werden.

Die Reaktionsbedingungen entsprechen bei dem erfindungsgemäßen Verfahren bezüglich Druck und Temperatur jenen, die aus dem Stand der Technik bekannt sind. So liegt die Reaktionstemperatur im allgemeinen im Bereich von 0 bis 90°C, bevorzugt wird ein Temperaturbereich von 20 bis 50 °C. Der Druck liegt im allgemeinen im Bereich von Atmosphärendruck oder geringem Unterdruck bis etwa 10 MPa. Vorzugsweise wird die Umsetzung bei einem Druck im Bereich von 0,5 bis 5 MPa durchgeführt.

In Gegenwart von Schwefelsäurealkylester zeigt der Edelmetallkatalysator eine hohe und lange anhaltende Aktivität für die Bildung von Wasserstoffperoxid mit einer erhöhten Selektivität der Wasserstoffperoxidbildung bezogen auf eingesetzten Wasserstoff, auch bei Verwendung von Wasserstoff und Sauerstoff enthaltenden Gasgemischen, die eine Zusammensetzung außerhalb des explosionsfähigen Bereichs aufweisen. Dabei können Reaktionsmedien eingesetzt werden, die geringere Gehalte an Halogeniden, wie Bromid und/oder Iodid, sowie an starken Säuren enthalten, als bei Abwesenheit von Schwefelsäurealkylester.

Das erfindungsgemäße Verfahren lässt sich in besonders vorteilhafter Weise in ein Gesamtverfahren zur Oxidation eines organischen Substrats mit Wasserstoffperoxid integrieren, da die mit dem erfindungsgemäßen Verfahren hergestellten Wasserstoffperoxidlösungen geringere Mengen an Komponenten enthalten, die bei einer nachfolgenden Oxidationsreaktion zu unerwünschten Nebenreaktionen und Folgereaktionen führen können. Besonders vorteilhaft sind dabei nach dem erfindungsgemäßen Verfahren hergestellte Lösungen von Wasserstoffperoxid in Methanol, die von 2 bis 15 Gew.-% und vorzugsweise von 5 bis 12 Gew.-% Wasserstoffperoxid, von 0,5 bis 20 Gew.-% und vorzugsweise von 2 bis 10 Gew.-% Wasser, von 60 bis 95 Gew.-% Methanol, von 10⁻⁶ bis 10⁻² mol/l und vorzugsweise von 10⁻⁵ bis 10⁻³ mol/l Bromid, sowie von 10⁻⁶ mol/l bis 0,1 mol/l und vorzugsweise von 0,001 mol/l bis 0,1 mol/l Dimethylsulfat und/oder Monomethylsulfat enthalten. Die Lösungen können zusätzlich noch bis zu 0,1 mol/l einer starken Säure, vorzugsweise Schwefelsäure enthalten.

Die erfindungsgemäßen Lösungen von Wasserstoffperoxid in Methanol eignen sich insbesondere zur Epoxidierung von Olefinen, vorzugsweise von Propen, mit Wasserstoffperoxid in Gegenwart eines titanhaltigen Zeoliths als Katalysator. Die erfindungsgemäßen Lösungen von Wasserstoffperoxid in Methanol können dabei ohne weitere Behandlung oder Zusätze in die Reaktion eingesetzt werden und ergeben im Vergleich zu nach dem Stand der Technik durch Direktsynthese hergestellten Lösungen von Wasserstoffperoxid in Methanol eine verbesserte Selektivität der Epoxidbildung und weniger Nebenprodukte durch Ringöffnungsreaktionen. Bei Verwendung von Lösungen, die zusätzlich eine starke Säure enthalten, wird der Gehalt an starker Säure vor der Verwendung zur Epoxidierung vorteilhaft durch Zusatz einer Base, vorzugsweise von Ammoniak, ganz oder teilweise neutralisiert. Die Epoxidierung kann unter den aus dem Stand der Technik, beispielsweise aus EP-A 0 100 119, bekannten Reaktionsbedingungen und mit den bekannten titanhaltigen Zeolithkatalysatoren durchgeführt werden. Vorzugsweise wird die Epoxidierung unter den in WO 02/085873 auf Seite 6, Zeile 17 bis Seite 11, Zeile 26 beschriebenen Reaktionsbedingungen durchgeführt, wobei die erfindungsgemäße Lösung von Wasserstoffperoxid in Methanol an Stelle der in WO 02/085873 verwendeten wässrigen Wasserstoffperoxidlösungen eingesetzt wird.

Die Erfindung wird durch die nachfolgenden Beispiele und Vergleichsbeispiele verdeutlicht.

### Beispiele:

### Beispiel 1

Die Direktsynthese wurde in einem Blasensäulenreaktor mit 16 mm Innendurchmesser und 40 cm Länge durchgeführt. Der Reaktor enthielt ein Katalysatorfestbett mit einem Schüttungsvolumen von etwa 80 ml. Als Katalysator wurde eine Mischung aus katalytisch aktiven Metallpartikeln und inerten Partikeln eingesetzt. Katalytisch wirksame Partikel aus 95 % Pd und 5 % Au wurden in Analogie zu DE 199 12 733 hergestellt. Als Inertmaterial wurde granulatförmiges alpha-Aluminiumoxid der Firma Ceramtech AG mit der Bezeichnung "Stemalox-Sprühkorn, gebrannt 0 - 0,5 mm Al₂O₃-Gehalt 85%" verwendet. Vor dem Einsatz wurde das Granulat durch Sieben von der Fraktion < 0,1 mm befreit. Das sehr feinteilige metallische Katalysatorpulver wurde mit dem oxidischen Trägerpulver gemischt. Der Pd-Gehalt der Mischung aus Katalysator- und inerten Partikeln betrug 0,25 Gew.-%.

Der Reaktor wurde als gefluteter Blasensäulenreaktor mit Gleichstrom von Gas und Flüssigkeit bei einem Druck von 5 MPa (50 bar) und einer Reaktionstemperatur von 25 °C betrieben. Als flüssiges Reaktionsmedium wurde ein Gemisch aus 98 Gewichtsteilen Methanol und 2 Gewichtsteilen Wasser mit 0,0001 mol/l Natriumbromid, 0,01 mol/l Schwefelsäure und 0,01 mol/l Dimethylsulfat verwendet. Das flüssige Reaktionsmedium wurde am unteren Ende des Reaktors mit 120 ml/h zugeführt. Aus der Strömungsgeschwindigkeit der Flüssigkeit und dem Reaktorquerschnitt ergab sich eine Flüssigkeitsquerschnittsbelastung von 0,6 m/h. Gleichzeitig wurden am unteren Ende des Reaktors 230 Nl/h einer Gasmischung aus 3 Vol.-% Wasserstoff, 20 Vol.-% Sauerstoff, sowie 77 Vol.-% Stickstoff eingespeist. Am oberen Ende des Reaktors wurde die gebildete Wasserstoffperoxidlösung zusammen mit nicht umgesetztem Gas so abgenommen, dass sich innerhalb des Reaktors kein Gaspolster ausbildete.

Nach 24 h Betrieb des Reaktors lag der Wasserstoffumsatz bei 49% und die entnommene Wasserstoffperoxidlösung enthielt 3,8 Gew.-% Wasserstoffperoxid. Die Selektivität der Wasserstoffperoxidbildung lag bei 71 % bezogen auf umgesetzten Wasserstoff. Die Katalysatoraktivität lag bei 10,6 g H₂O₂ / g Pd * h.

### Beispiel 2 (nicht erfindungsgemäß)

Beispiel 1 wurde wiederholt, dem Reaktionsmedium wurde jedoch kein Dimethylsulfat zugesetzt.

Nach 24 h Betrieb des Reaktors lag der Wasserstoffumsatz bei 45% und die entnommene Wasserstoffperoxidlösung enthielt 3,12 Gew.-% Wasserstoffperoxid. Die Selektivität der Wasserstoffperoxidbildung lag bei 64 % bezogen auf umgesetzten Wasserstoff. Die Katalysatoraktivität lag bei 8,5 g H₂O₂ / g Pd * h.

### Beispiel 3 (nicht erfindungsgemäß)

Beispiel 2 wurde wiederholt, die Konzentration an Schwefelsäure wurde jedoch auf 0,001 mol/l verringert.

Nach 24 h Betrieb des Reaktors lag der Wasserstoffumsatz bei 45% und die entnommene Wasserstoffperoxidlösung enthielt 2,25 Gew.-% Wasserstoffperoxid. Die Selektivität der Wasserstoffperoxidbildung lag bei 46 % bezogen auf umgesetzten Wasserstoff. Die Katalysatoraktivität lag bei 6,1 g H₂O₂ / g Pd * h.

### Beispiel 4

Beispiel 1 wurde wiederholt, dem Reaktionsmedium wurde jedoch keine Schwefelsäure zugesetzt und die Konzentration an Dimethylsulfat wurde auf 0,02 mol/l erhöht.

Nach 24 h Betrieb des Reaktors lag der Wasserstoffumsatz bei 49% und die entnommene Wasserstoffperoxidlösung enthielt 4,0 Gew.-% Wasserstoffperoxid. Die Selektivität der Wasserstoffperoxidbildung lag bei 74 % bezogen auf umgesetzten Wasserstoff. Die Katalysatoraktivität lag bei 11,0 g H₂O₂ / g Pd * h. Katalysatoraktivität und Selektivität der Wasserstoffperoxidbildung waren auch nach 72 h Betrieb des Reaktors unverändert.

Ein Vergleich der Beispiele 1 und 2 zeigt, dass in Gegenwart von Dimethylsulfat eine höhere Aktivität und Selektivität des Katalysators für die Bildung von Wasserstoffperoxid erreicht wurde als bei sonst gleichen Bedingungen ohne Zusatz von Dimethylsulfat.

Beispiel 3 zeigt im Vergleich zu Beispiel 2, dass in Abwesenheit eines Schwefelsäurealkylesters die Selektivität der Wasserstoffperoxidbildung bei verringerter Säurekonzentration stark abnimmt. Beispiel 4 zeigt dagegen, dass in Gegenwart von Dimethylsulfat auch ohne Zusatz einer starken Säure eine hohe Selektivität der Wasserstoffperoxidbildung erzielt wurde.

## Patentansprüche

1. Verfahren zur Herstellung von Wasserstoffperoxid durch Reaktion von Wasserstoff und Sauerstoff in Gegenwart eines Edelmetallkatalysators in einem flüssigen Reaktionsmedium,
**dadurch gekennzeichnet,**
**dass** die Reaktion in Gegenwart eines Schwefelsäurealkylesters durchgeführt wird.

2. Verfahren nach Anspruch 1,
**dadurch gekennzeichnet,**
**dass** der Schwefelsäurealkylester ein Schwefelsäuredialkylester, ein Schwefelsäuremonoalkylester oder ein Alkalimetall- oder Ammoniumsalz eines Schwefelsäuremonoalkylesters ist.

3. Verfahren nach Anspruch 2,
**dadurch gekennzeichnet,**
**dass** der Alkylrest des Schwefelsäurealkylesters aus der Gruppe Methyl, Ethyl, n-Propyl und n-Butyl gewählt wird und vorzugsweise Methyl ist.

4. Verfahren nach einem der Ansprüche 1 bis 3,
**dadurch gekennzeichnet,**
**dass** die Reaktion in einem wässrigen, wässrig-organischen oder organischen Reaktionsmedium durchgeführt wird, das ein Bromid und/oder Iodid gelöst enthält.

5. Verfahren nach Anspruch 4,
**dadurch gekennzeichnet,**
**dass** das wässrig-organische oder organische Reaktionsmedium ein Lösungsmittel aus der Reihe Methanol, Ethanol, n-Propanol und n-Butanol, vorzugsweise Methanol, enthält.

6. Verfahren nach einem der vorangehenden Ansprüche,
**dadurch gekennzeichnet,**
**dass** die Reaktion mit einem Wasserstoff und Sauerstoff enthaltenden, nicht-explosiven Gasgemisch durchgeführt wird.

7. Verfahren nach einem der vorangehenden Ansprüche,
**dadurch gekennzeichnet,**
**dass** der Edelmetallkatalysator metallisches Palladium auf einem Trägermaterial enthält.

8. Verfahren nach einem der vorangehenden Ansprüche,
**dadurch gekennzeichnet,**
**dass** die Reaktion in einem Reaktor durchgeführt wird, in dem der Edelmetallkatalysator in Form von einem oder mehreren Festbetten angeordnet ist und ein Wasserstoff und Sauerstoff enthaltendes Gasgemisch so durch den Reaktor geleitet wird, dass die Oberfläche des Reaktors an keiner Stelle dauerhaft in Kontakt mit dem durch den Reaktor geleiteten Gasgemisch steht.

9. Wasserstoffperoxidlösung, enthaltend
von 2 bis 15 Gew.-% Wasserstoffperoxid,
von 0,5 bis 20 Gew.-% Wasser,
von 60 bis 95 Gew.-% Methanol,
von 10⁻⁶ bis 10⁻² mol/l Bromid und
von 10⁻⁶ bis 0,1 mol/l Dimethylsulfat und/oder Monomethylsulfat.

10. Verfahren zur Herstellung von Epoxiden durch Umsetzung eines Olefins mit Wasserstoffperoxid in Gegenwart eines titanhaltigen Zeoliths als Katalysator,
**dadurch gekennzeichnet,**
**dass** Wasserstoffperoxid in Form einer Lösung gemäß Anspruch 9 eingesetzt wird.

## Claims

1. Process for producing hydrogen peroxide by reacting hydrogen and oxygen in the presence of a noble metal catalyst in a liquid reaction medium,
**characterised in that**
the reaction is performed in the presence of a sulfuric acid alkyl ester.

2. Process according to claim 1,
**characterised in that**
the sulfuric acid alkyl ester is a sulfuric acid dialkyl ester, a sulfuric acid monoalkyl ester or an alkali metal or ammonium salt of a sulfuric acid monoalkyl ester.

3. Process according to claim 2,
**characterised in that**
the alkyl radical in the sulfuric acid alkyl ester is chosen from the group of methyl, ethyl, n-propyl and n-butyl and is preferably methyl.

4. Process according to one of claims 1 to 3,
**characterised in that**
the reaction is performed in an aqueous, aqueous-organic or organic reaction medium containing a bromide and/or iodide in dissolved form.

5. Process according to claim 4,
**characterised in that**
the aqueous-organic or organic reaction medium comprises a solvent from the series of methanol, ethanol, n-propanol and n-butanol, preferably methanol.

6. Process according to one of the preceding claims,
**characterised in that**
the reaction is performed with a non-explosive gas mixture containing hydrogen and oxygen.

7. Process according to one of the preceding claims,
**characterised in that**
the noble metal catalyst comprises metallic palladium on a support material.

8. Process according to one of the preceding claims,
**characterised in that**
the reaction is performed in a reactor in which the noble metal catalyst is arranged in the form of one or more fixed beds and a gas mixture containing hydrogen and oxygen is passed through the reactor in such a way that at no point is the surface of the reactor permanently in contact with the gas mixture being passed through the reactor.

9. Hydrogen peroxide solution, comprising
2 to 15 wt.% hydrogen peroxide,
0.5 to 20 wt.% water,
60 to 95 wt.% methanol,
10⁻⁶ to 10⁻² mol/l bromide and
10⁻⁶ to 0.1 mol/l dimethyl sulfate and/or monomethyl sulfate.

10. Process for producing epoxides by reacting an olefin with hydrogen peroxide in the presence of a titanium-containing zeolite as catalyst,
**characterised in that**
hydrogen peroxide is used in the form of a solution according to claim 9.

## Revendications

1. procédé pour la production de peroxyde d'hydrogène par la réaction d'hydrogène et d'oxygène en présence d'un catalyseur contenant un métal noble, dans un milieu réactionnel liquide, **caractérisé en ce que** la réaction est effectuée en présence d'un sulfate d'alkyle.

2. Procédé selon la revendication 1, **caractérisé en ce que** le sulfate d'alkyle est un sulfate de dialkyle, un sulfate de monoalkyle ou un sel de métal alcalin ou d'ammonium d'un sulfate de monoalkyle.

3. Procédé selon la revendication 2, **caractérisé en ce que** le fragment alkyle du sulfate d'alkyle est choisi dans l'ensemble constitué par les groupes méthyle, éthyle, n-propyle et n-butyle, et de préférence est le groupe méthyle.

4. Procédé selon l'une quelconque des revendications 1 à 3, **caractérisé en ce que** la réaction est effectuée dans un milieu réactionnel aqueux, aqueux-organique ou organique qui contient un bromure et/ou un iodure dissous.

5. Procédé selon la revendication 4, **caractérisé en ce que** le milieu réactionnel aqueux-organique ou organique contient un solvant choisi parmi le méthanol, l'éthanol, le n-propanol et le n-butanol, de préférence qui est le méthanol.

6. Procédé selon l'une quelconque des revendications précédentes, **caractérisé en ce que** la réaction est effectuée avec un mélange gazeux non explosif contenant de l'hydrogène et de l'oxygène.

7. Procédé selon l'une quelconque des revendications précédentes, **caractérisé en ce que** le catalyseur contenant un métal noble contient du palladium métallique sur une matière de support.

8. Procédé selon l'une quelconque des revendications précédentes, **caractérisé en ce que** la réaction est effectuée dans un réacteur dans lequel le catalyseur contenant un métal noble est disposé sous forme d'un ou plusieurs lits fixes et on fait passer dans le réacteur un mélange gazeux contenant de l'hydrogène et de l'oxygène, de sorte que la surface du réacteur n'est en aucun point durablement en contact avec le mélange gazeux envoyé dans le réacteur.

9. Solution de peroxyde d'hydrogène, contenant
de 2 à 15 % en poids de peroxyde d'hydrogène,
de 0,5 à 20 % en poids d'eau,
de 60 à 95 % en poids de méthanol,
de 10⁻⁶ à 10⁻² mole/l de bromure et
de 10⁻⁶ à 0,1 mole/l de sulfate de diméthyle et/ou de sulfate de monométhyle.

10. Procédé pour la préparation d'époxydes par la mise en réaction d'une oléfine avec du peroxyde d'hydrogène en présence d'une zéolithe contenant du titane, en tant que catalyseur, **caractérisé en ce que** le peroxyde d'hydrogène est utilisé sous forme d'une solution selon la revendication 9.
